# EUROPEAN PATENT SPECIFICATION

(11) **EP 3 251 599 B1**
(45) Date of publication and mention of the grant of the patent: **30.12.2020**
(21) Application number: 15879713.4
(22) Date of filing: 23.11.2015
(51) Int. Cl.: A61B 6/03, A61B 6/00, A61B 6/14, A61B 6/06

(54) **METHOD FOR CAPTURING TOMOGRAPHIC IMAGE BY A COMPUTER TOMOGRAPHY APPARATUS**
COMPUTERTOMOGRAFIEVERFAHREN ZUR AUFNAHME EINES TOMOGRAFISCHEN BILDES MIT EINER COMPUTERTOMOGRAFIEVORRICHTUNG
PROCÉDÉ D'ACQUISITION D'IMAGE TOMOGRAPHIQUE UTILISANT UN DISPOSITIF DE TOMOGRAPHIE ASSISTÉE PAR ORDINATEUR

(30) Priority: 30.01.2015 CN 201510050900
(43) Date of publication of application: 06.12.2017
(73) Proprietor: Hefei Meyer Optoelectronic Technology Inc., Hefei, Anhui 230088 (CN)
(72) Inventor: TIAN, Ming, Hefei Anhui 230088 (CN); LIN, Maoxian, Hefei Anhui 230088 (CN); JIANG, Dong, Hefei Anhui 230088 (CN); WANG, Yu, Hefei Anhui 230088 (CN); FANG, Yong, Hefei Anhui 230088 (CN); ZHAO, Youyuan, Hefei Anhui 230088 (CN); ZHANG, Jianjun, Hefei Anhui 230088 (CN)
(74) Representative: advotec.
(86) International application number: PCT/CN2015/095342
(87) International publication number: WO 2016/119515

(56) References cited:
- EP-A1- 2 659 838
- EP-A1- 2 730 227
- WO-A1-2014/037770
- WO-A1-2014/069186
- WO-A2-2008/028058
- WO-A2-2012/032220
- CN-A- 1 293 532
- CN-A- 1 794 950
- CN-A- 102 258 379
- CN-U- 202 699 160
- JP-A- 2009 118 925
- US-A- 6 118 842
- US-A1- 2008 232 540
- US-A1- 2009 168 966
- Pau Montes: "Dynamic Cone-beam Reconstruction for Perfusion Computed Tomography", , 6 December 2006 (2006-12-06), XP055540706, Retrieved from the Internet: URL:http://archiv.ub.uni-heidelberg.de/vol ltextserver/7020/1/Dissertation_PauMontes. pdf [retrieved on 2019-01-11]

## Description

### FIELD

The present disclosure relates to a field of medical technology, and more particularly relates to a computed tomography method for capturing tomographic image.

### BACKGROUND

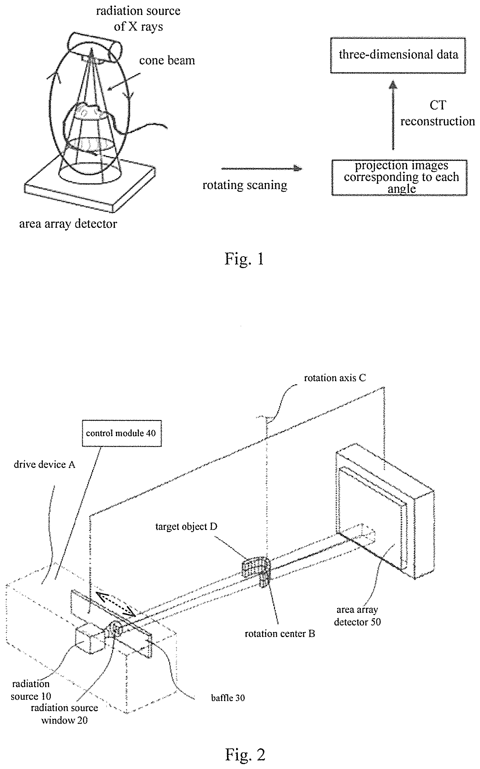

Oral CBCT (Cone beam CT) is a process to reconstruct an object in a field of view. As shown in Fig. 1, basic principles of CBCT is: rotationally scanning a checking object by using an area array detector and a cone beam X-ray radiation source to acquire two-dimensional projection images of the checking object from various angles, and then acquiring three-dimensional data inside the checking object according to a CBCT reconstruction algorithm.

At present, available oral CBCT is usually an apparatus for capturing a panoramic image of an object. A user may have to view a partial image corresponding to a single tooth from the panoramic image acquired if the single tooth is only required to be checked, which may lead to waste of resources, such as an improvement of radiation dose. Furthermore, from a perspective of clarity, due to a size limit of the partial image, it is difficult to view the partial image from the panoramic image, which means that the resolution of the partial image may be reduced

There are also available imaging systems configured to reconstruct an image of a region of interest indicated on a panorama image as disclosed in publication US 2009/0168966 A1.

### SUMMARY

Embodiments of the present disclosure seek to solve at least one of the problems existing in the related art to at least some extent.

There is described a computed tomography apparatus not forming part of the invention. The computed tomography apparatus may be configured to control a baffle such that a through-hole in the baffle is arranged in a center of a radiation source window, and to determine a photographing position of a partial area by controlling a rotation center, so as to realize CT photographing for the partial area of an object to be captured. Thus, compared to CT photographing for the whole area of an object to be captured, photographing time may be saved, radiation dosage may be reduced, and resolution of a partial image may be improved from a respective of definition.

The present invention provides a method for capturing a tomographic image as defined in the claims. The computed tomography apparatus includes: a radiation source, configured to emit X rays; a radiation source window; a baffle, having a through-hole therein; a control module, connected to the baffle via a drive device, configured to control a rotation center of a rotation arm of the computed tomography apparatus according to a preset photographing condition to determine a photographing position of an image to be captured, and to regulate the baffle via the drive device according to a position of a partial area of a target object when the photographing position is the partial area of the target object, such that the X rays pass through the radiation source window and penetrate through the through-hole and the partial area to an area array detector; and the area array detector, configured to convert the received X rays penetrating through the through-hole and the partial area to a projection image.

The through-hole which can be regulated may be set in the baffle, the rotation center of the rotation arm in the computed tomography apparatus may be controlled by the control module to determine the photographing position to be captured, and the baffle may be regulated via the drive device according to the position of the partial area to perform CT photographing on the target object when the photographing position to be captured is the partial area of the target object. For example, when a user only needs to observe a partial area of the object to be captured, the through-hole is arranged in a center of the radiation source window by controlling the baffle, and the photographing position of the partial area may be determined by controlling the rotation center, such that the CT photographing may be performed on the partial area of the object to be captured. Thus, compared to the CT photographing for a whole area of the object to be captured, photographing time may be saved, radiation dosage may be reduced, and resolution of a partial image may be improved from a respective of definition.

In order to achieve the above objectives, the present disclosure provide a method for capturing a tomographic image by the computed tomography apparatus provided in the present disclosure. The method includes: acquiring a pre-captured image; receiving a selecting instruction for the pre-captured image from a user, and determining an area of an object to be captured according to the selecting instruction; determining a position on X direction of the rotation center, a position on Y direction of the rotation center, and a height range of photographing view on Z direction, such that the computed tomography apparatus captures the area according to the position on X direction, the position on Y direction, and the height range of photographing view on Z direction; and acquiring the projection image captured, and performing a CT reconstruction on the projection image according to a preset CT reconstruction model to generate a three-dimensional image.

According to the method the selecting instruction for the pre-captured image are received from the user, the area of the object to be captured is determined according to the selecting instruction, the position on X direction and the position on Y direction of the rotation center of the rotation arm in the computed tomography apparatus and the height range of photographing view on Z direction are determined according to the area, such that the computed tomography apparatus captures the area according to the position on X direction and the position on Y direction of the rotation center, and the height range of photographing view on Z direction, the projection image captured is acquired, and a CT reconstruction is performed on the projection image according to the preset CT reconstruction model to generate the three-dimensional image. For example, when the user only needs to observe a partial area of the object to be captured, the baffle can be regulated by the computed tomography apparatus to realize CT photographing for a partial area of the target object. Further, for example, when the user only needs to observe a whole area of the object to be captured, the baffle is regulated by the computed tomography apparatus to realize CT photographing for a whole area of the target object, i.e., the baffle is regulated by the computed tomography apparatus to switch between the CT photographing for a partial area and the CT photographing for a whole area.

Additional aspects and advantages of embodiments of present disclosure will be given in part in the following descriptions, become apparent in part from the following descriptions, or be learned from the practice of the embodiments of the present disclosure.

### BRIEF DESCRIPTION OF THE DRAWINGS

These and/or other aspects and advantages of embodiments of the present disclosure will become apparent and more readily appreciated from the following descriptions made with reference to the accompanying drawings, in which:
Fig. 1 is a schematic diagram of a fundamental principle of oral CBCT;
Fig. 2 is a structure diagram of a computed tomography apparatus according to one embodiment of the present disclosure;
Fig. 3 is a diagram of a structure between a radiation source window and a through-hole in a baffle based on the computed tomography apparatus as shown in Fig. 2;
Fig. 4 is a diagram of a relationship between a position of the radiation source window and a position of the baffle when CT photographing for a partial area is performed according to one embodiment of the present disclosure;
Fig. 5 is a flow chart of a method for capturing a tomographic image according to one embodiment of the present disclosure;
Fig. 6 is a schematic diagram of positions of frontal projections and lateral projections according to embodiments of the present disclosure;
Fig. 7 is a schematic diagram illustrating imaging of a left target of teeth and a right target of teeth according to embodiments of the present disclosure;
Fig. 8 is a schematic diagram of a pre-captured image according to embodiments of the present disclosure;
Fig. 9 is a schematic diagram of different projection areas on a detector corresponding to different photographing views according to embodiments of the present disclosure;
Fig. 10 is a schematic diagram of a full view three-dimensional image after a reconstruction when CT photographing for a whole area is performed;
Fig. 11 is a schematic diagram of a partial view three-dimensional image after a reconstruction when CT photographing for a partial area is performed; and
Fig. 12 is a diagram of a relationship between a position of the radiation source, a position of the detector, and the object to be captured in a process of photographing.

### DETAILED DESCRIPTION

Reference will be made in detail to embodiments of the present disclosure, where the same or similar elements and the elements having same or similar functions are denoted by like reference numerals throughout the descriptions. The embodiments described herein with reference to drawings are explanatory, illustrative, and used to generally understand the present disclosure. The embodiments shall not be construed to limit the present disclosure.

The computed tomography apparatus and the method for capturing a tomographic image by the computed tomography apparatus according to embodiments of the present disclosure will be described with reference to drawings.

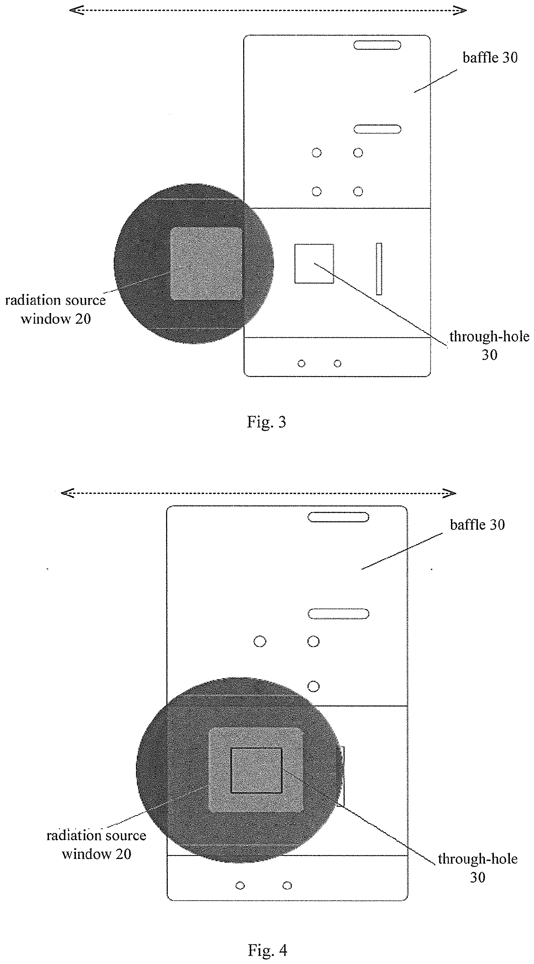

Fig. 2 is a structure diagram of a computed tomography apparatus according to one embodiment of the present disclosure. Fig. 3 is a diagram of a structure between a radiation source window and a through-hole in a baffle based on the computed tomography apparatus as shown in Fig. 2. The through-hole in the baffle is not illustrated in Fig. 2, and a radiation source, a control module and an area array detector are not illustrated in Fig. 3. It should be noted that the computed tomography apparatus according to embodiments of the present disclosure may be applied on a computed tomography CT machine, and more particularly, may be applied on an oral CBCT. It may be take an example that the computed tomography apparatus is applied on the oral CBCT.

As shown in Fig. 2 and Fig. 3, the computed tomography apparatus may include a radiation source 10, a radiation source window 20, a baffle 30, a control module 40 and an area array detector 50. In embodiments of the present disclosure, as shown in Fig. 3, a window (may be called as a CT window) may be provided in the radiation source window 20, and all parts of the radiation source window except for the window are covered by a lead plate to avoid X rays penetrating through any part of the radiation source window except the window to affect a photographing result. Additionally, as shown in Fig. 2 and Fig. 3, the baffle 30 may have the through-hole 31 therein. In the embodiments of the present disclosure, a size of the through-hole 31 is smaller than a size of the radiation source window 20.

Specifically, the radiation source 10 may be configured to emit the X rays.

The control module 40 may be connected to the baffle 30 via a drive device A. The control module 40 may be configured to control a rotation center B of a rotation arm of the computed tomography apparatus mentioned above according to a preset photographing condition so as to determine a photographing position to be captured. The baffle 30 may be regulated via the drive device A according to a position or an angle of a partial area of a target object D when the photographing position is the partial area of the target object D, such that the X rays pass through the radiation source window 20 and penetrate through the through-hole 31 and the partial area to an area array detector 50. In the embodiments of the present disclosure, the preset photographing condition may include but is not limited to a requirement of resolution of partial tomography etc.. It should be noted that, in the embodiments of the present disclosure, a vertical axis which is perpendicular to a supporting arm of a C-sharped arm (such as the rotation arm mentioned above) and the C-sharped arm rotates around is called as a rotation axis C, and an intersection point of the rotation axis C and a horizontal section of the target object D (such as teeth) is called as the rotation center B.

Specifically, in the embodiments of the present disclosure, the control module 40 may regulate the baffle 30 via the drive device A according to the partial area mentioned above, such that the baffle 30 may move to the radiation source window 20 and allow the X rays passing through the radiation source window 20 and penetrating through the through-hole 31 and the partial area to the area array detector 50. The control module 40 may be configured to move the baffle in a left and right direction via the drive device, so as to regulate an amount of X rays passing though the through-hole 31. In other words, the control module 40 may be configured to adjust the position to be captured by moving the rotation center B all around, and to control the through-hole 31 moving to a center of the radiation source window 20 by moving the baffle 30 right and left according to the determined position to be captured, such that X rays pass through the radiation source window 20, penetrate through the through-hole 31 and the object to be captured, and are emitted on the area array detector 50.

The area array detector 50 is configured to convert the received X rays penetrating through the through-hole 31 and the partial area mentioned above to a projection image, such that a computer connected to the computed tomography apparatus performs a CT reconstruction on the projection image to generate a partial view three-dimensional image. In the embodiments of the present disclosure, the area array detector 50 may be an indirect conversion flat panel detector based on CMOS technology, and a scintillator in the indirect conversion flat panel detector has cesium iodide.

Further, in one embodiment of the present disclosure, when the determined photographing position to be captured is a whole area of the target object, the control module 40 is further configured to drive the baffle 30 away from the radiation source window 20 via the driving device A according to the whole area of the target object D, such that the X rays penetrate through the radiation source window 20 and the whole area of the target object D to the area array detector 50. The area array detector 50 is further configured to convert the received X rays which penetrate through the radiation source window 20 and the whole area of the target object D to the projection image, such that the computer connected to the computed tomography apparatus performs a CT reconstruction on the projection image to generate a full view three-dimensional image. In the embodiments of the present disclosure, driving the baffle 30 away from the radiation source window 20 via the driving device A can be understood as moving the baffle 30 in the left and right direction via the drive device A, such that the baffle 30 doesn't cover the radiation source window 20, as shown in Fig. 2.

More particularly, the control module 40 is configured to adjust the position to be captured (such as the whole area of teeth to be captured) by moving the rotation center B all around, and to control the baffle 30 away form (i.e., not cover) the radiation source window 20 by moving the baffle 30 all around via the driving device A according to the determined position to be captured (such as the whole area of teeth to be captured). In other words, the baffle 30 may be controlled to not block the X rays from passing through the radiation source window 20, i.e., to allow the X rays to passing through the radiation source window 20 to the area array detector 50.

In order to allow those skilled in the art to understand the present disclosure, principles of the computed tomography apparatus when photographing will be described as follows.

It could be understood that, the control module 40 may control the rotation center B of the rotation arm of the computed tomography apparatus and the baffle 30 via the drive device A (such as a power system in the computed tomography apparatus, i.e., a motor), to realize CT photographing for a whole area and CT photographing for a partial area of the target object. The process to realize this will be described as follows.

Fig. 3 also illustrates a relationship between a position of the radiation source window 20 and a position of the baffle 30 when the CT photographing for a whole area is performed. As shown in Fig. 3, the control module 40 may control the baffle 30 to move in the left and right direction via the drive device, such that the baffle 30 doesn't cover the radiation source window 20 and the X rays can pass the radiation source window 20 directly, thus realizing the CT photographing for a whole area, in which the radiation source window 20 can be called as a CT window.

Fig. 4 illustrates a relationship between a position of the radiation source window 20 and a position of the baffle 30 when the CT photographing for a partial area is performed according to an embodiment of the present disclosure. The control module 40 may control the baffle 30 to move in the left and right direction via the drive device until the baffle 30 reaches the radiation source window 20, such that the baffle 30 covers the X rays passing through the radiation source window 20, which means that the X rays may be emitted through the through-hole 31 in the baffle 30, thus realizing the CT photographing for a partial area of the photographing device. It could be understood that the size of the through-hole 31 is smaller than the size of the radiation source window 20. For example, a height of a partial CT view is 4 CM, while a height of a CT view of a machine is 8 CM, then a height of the through-hole 31 in the baffle 30 is just desired to be 1/2 of a height of the radiation source window 20.

Specifically, the through-hole 31 of which an opening can be regulated is provided in the baffle 30. The control module 40 may regulate the size of the through-hole 31 and determine the photographing position according to the preset photographing condition (such as a requirement of resolution of a partial CT). Specifically, the control module 40 may move the rotation center B to adjust and determine the photographing position to be captured. A distance between any two of the radiation source 10, the through-hole 31 and the area array detector 50 is adjusted according to a requirement of image resolution. A part of the baffle 30 may cover the radiation source window 20 by moving the baffle 30 in the left and right direction, which is similar to adjusting the size of the through hole 31 in a case that the distance between any two of the radiation source 10, the through-hole 31 and the area array detector 50 is not changed, , such that the X rays emitted by the radiation source 10 pass through the radiation source window 20 and penetrate through the through-hole 31 in the baffle 30 to the area array detector 50. The area array detector 50 may convert the received X rays to the projection image. The computer connected to the computed tomography apparatus performs a CT reconstruction on the projection images corresponding to each angle to generate three-dimensional data of the object captured. More particularly, the radiation source 10 may be approximated as a point light source, thus an area of the projection image converted by the area array detector 50 may be computed in an equal ratio way, such that a size of the three-dimensional image may be acquired.

It could be seen that the radiation source window 20 is the CT window when the baffle 30 doesn't cover the radiation source window 20. The baffle 30 may be made of a lead plate and the through-hole 31 in the baffle 30 may be a partial CT window. The CT photographing for a partial area can be realized just by moving the position of the baffle 30 by the control module 40 such that the through-hole 31 is located in the center of the radiation source window 20 when photographing.

In addition, it is familiar to those skilled in the art that the area array detector 50 is one of core components of CBCT, which mainly has two types: X ray Image Intensifier (I.I. for short) and Flat Panel Detector (FPD for short). At present, most of the CBCT systems apply FPD. This is because FPD has higher space resolution, higher construct ratio and longer using service life, and because FPD avoids edge distortion. Flat panel detector will be described as follows.

According to an energy conversion mode, there are two kinds of flat panel detectors: an indirect conversion flat panel detector and a direct conversion flat panel detector. The indirect conversion FPD applies scintillator as a conversion material. Firstly, the X rays are converted to a visible light, and then a final electrical signal is acquired by capturing and measuring photons of the visible light. There are mainly two kinds of large area scintillators: cesium iodide (CsI) and gadolinium oxysulfide (Gd2O2s). CsI is more often chosen as a scintillator in medical imaging, because CsI has better conversion efficiency in a low energy X ray segment.

According to different back-end circuits of the scintillator, the indirect conversion flat panel detector may be divided into a flat panel detector based on amorphous silicon (a-Si) technology and a flat panel detector based on CCD/CMOS technology. The flat panel based on a-Si technology mainly includes a CsI scintillator, a photoelectric diode matrix made of a-Si and a thin film transistor (TFT). Photons of the invisible X rays are absorbed by a CsI coating and are converted to photons of a visible light, and then the photons of the visible light are converted to electric charges via the photoelectric diode and introduced into the thin film transistor, and finally, the electric charges are output by a read-out circuit. An imaging principle of the flat panel detector based on CCD/CMOS is similar to that of the flat panel detector based on a-Si, however, a fill factor of an effective detecting area per unit is higher and stability of the system is better in the flat panel detector based on CCD/CMOS, because a more sophisticated low resolution CMOS/CCD technology is applied directly.

At present, the direct conversion FPD usually applies amorphous selenium technology which could be divided into two layers: an amorphous selenium semiconductor coating and a TFT array. Incident photons of the X rays are absorbed by the amorphous selenium coating and are converted to electronic charges directly. Under an outside electric field, the electric charges (i.e., electron-hole pairs) are accumulated in a pixel capacitor and are read out by the TFT finally. Compared to indirect conversion flat panel, imaging accuracy of the direct conversion flat panel is higher and is able to realize a point spread function with a width of only 1 µm. This is because the X rays are converted to the visible light by the indirect conversion flat panel, while there may be scattering and diffusion when the visible light is transmitted in the scintillator, which may reduce the image resolution. However, the direct conversion flat panel directly generates the electron-hole pairs, thus avoiding the spread of signals. Since a cost of the direct conversion flat panel is higher, the indirect conversion flat panel is applied more often in an oral clinical at present.

Thus, the area array detector 50 according to the embodiments of the present disclosure applies the indirect conversion flat, i.e., the flat panel detector based on CMOS technology and applying CsI as a large area scintillator.

According to the computed tomography apparatus of the embodiments of the present disclosure, the through-hole which can be regulated may be set in the baffle, the rotation center of the rotation arm of the computed tomography apparatus may be controlled by the control module to determine the photographing position to be captured, and the baffle may be regulated via the drive device according to the position of the partial area to perform the CT photographing on the target object when the photographing position to be captured is the partial area of the target object. For example, when a user only needs to observe the partial area of the object to be captured, the through-hole is arranged in the center of the radiation source window by controlling the baffle, and the photographing position of the partial area may be determined by controlling the rotation center, such that the CT photographing for a partial area may be performed on the object to be captured. Thus, compared to CT photographing for a whole area, photographing time may be saved, radiation dosage may be reduced, and resolution of a partial image may be improved from a respective of definition.

Additionally, the present disclosure provides a method for capturing a tomographic image by the computed tomography apparatus provided in any of the above embodiments of the present disclosure.

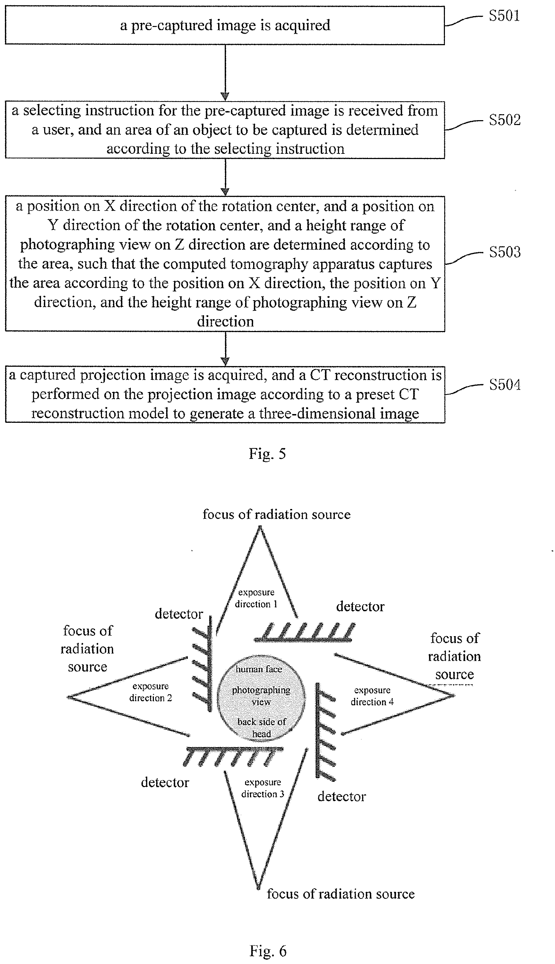

Fig. 5 is a flow chart of a method for capturing a tomographic image according to one embodiment of the present disclosure. As shown in Fig. 5, the method may include flowing acts.

In act S501, a pre-captured image is acquired.

It should be noted that, in the embodiments of the present disclosure, considering that the area array detector in the computed tomography apparatus may be deviated, the object to be captured (such as the teeth) may be divided into a left area and a right area. It should be understood that the object to be captured may not be divided into the left area and the right area if the area array detector is arranged opposite a center.

When the rotation center of the computed tomography apparatus is exactly opposite the center of the area array detector, it can be called that the area array detector is arranged opposite the center. When the rotation center is deviated to one side of the center of the area array detector, it can be called that the area array detector is deviated. When the area array detector is completely arranged at one side of the rotation center and can obtain a complete image of the target object, it can be called that the area array detector is totally deviated. It should be understood that when the rotation center is opposite the center, the rotation arm of the computed tomography apparatus (such as a CT machine) may be rotated more than 180° to perform a reconstruction. When the rotation center is deviated, the rotation arm of the computed tomography apparatus (such as a CT machine) may be rotated more than 360° to perform the reconstruction. When the rotation center is deviated, a minimum rotating angle (such as an angle between 180° and 360°) of the rotation arm is determined according to an amount of a deviated position. It could be understood that a vertical axis which is perpendicular to a supporting arm of a C-sharped arm (such as the rotation arm mentioned above) and the C-sharped arm rotates around is called as a rotation axis, and an intersection point of the rotation axis and a horizontal section of the teeth is called as the rotation center.

Additionally, the reason for dividing the left area and the right area when the area array detector is deviated is that one side may not be captured when a frontal projection is captured by using only a single exposure direction. As shown in Fig. 6, right teeth can not be captured totally when exposure direction 1 is used. Thus, exposure direction 3 is necessary for capturing the frontal projection of the right area.

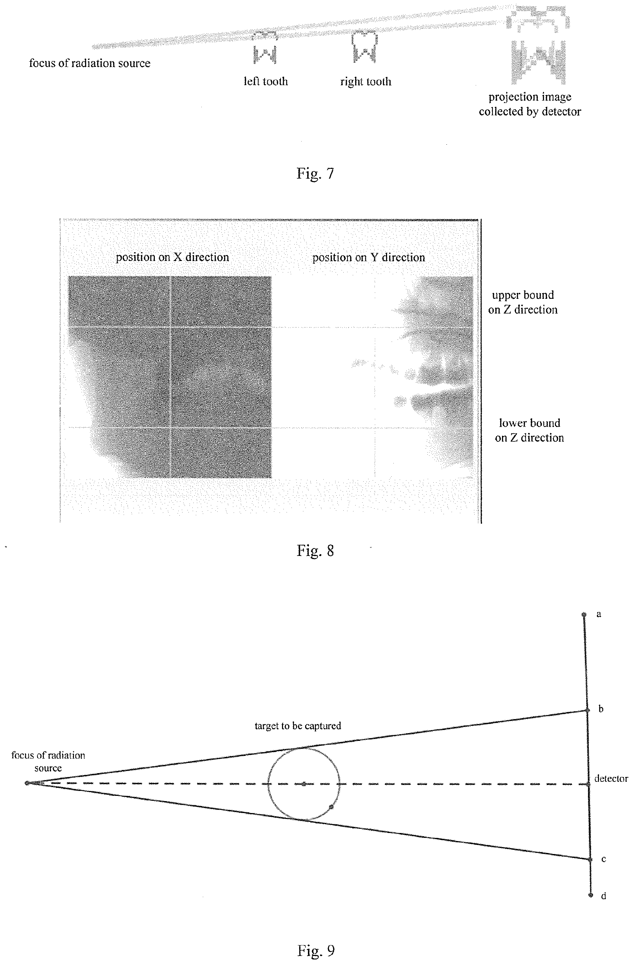

Further, Fig. 7 is a schematic diagram illustrating imaging of a left target of teeth and a right target of teeth according to embodiments of the present disclosure. From a perspective of magnification ratio, it could be seen from Fig. 6 and Fig. 7 that the projection image of the left area is larger than that of the right area by using exposure direction 2, thus a lateral projection of the left area is captured by using exposure direction 2. Similarly, a lateral projection of the right area is captured by using exposure direction 4.

Specifically, a pair of frontal projections and a pair of lateral projections are captured by the computed tomography apparatus firstly, i.e., capturing the pair of frontal projections is capturing one frontal projection by using exposure direction 1 and by using exposure direction 3 respectively, and the pair of lateral projections is captured in a similar way. For example, with a position of a human in Fig. 6, the projections captured by using exposure direction 1 and exposure direction 3 are the frontal projections, and the projections captured by using exposure direction 2 and exposure direction 4 are the lateral projections. If the area array detector is deviated according to directions in Fig. 6, a frontal projection of the left teeth is captured in exposure direction 1, a lateral projection of the left teeth is captured in exposure direction 2, a frontal projection of the right teeth is captured in exposure direction 3, and a lateral projection of the right teeth is captured in exposure direction 4. If the area array detector is deviated in directions opposite directions in Fig. 6, the frontal projection of the left teeth is captured in exposure direction 3, the lateral projection of the left teeth is captured in exposure direction 2, the frontal projection of the right teeth is captured in exposure direction 1, and the lateral projection of the right teeth is captured in exposure direction 4. For example, with the position of the human and the deviated directions of the area array detector in Fig. 6, the pre -captured image is obtained. As shown in Fig. 8, a left image in Fig. 8 is the frontal projection and a right image is the lateral projection.

In act S502, a selecting instruction for the pre-captured image is received from a user, and an area of an object to be captured is determined according to the selecting instruction.

Specifically, the user may select from the pre-captured image to select an area to be captured. For example, the user may select a position corresponding to the teeth to be captured by moving lines shown in Fig. 8, thus the area of the object to be captured is determined according to the selecting instruction of the user.

In act 503, a position on X direction of the rotation center, a position on Y direction of the rotation center, and a height range of photographing view on Z direction are determined according to the area, such that the computed tomography apparatus captures the area according to the position on X direction, the position on Y direction, and the height range of photographing view on Z direction.

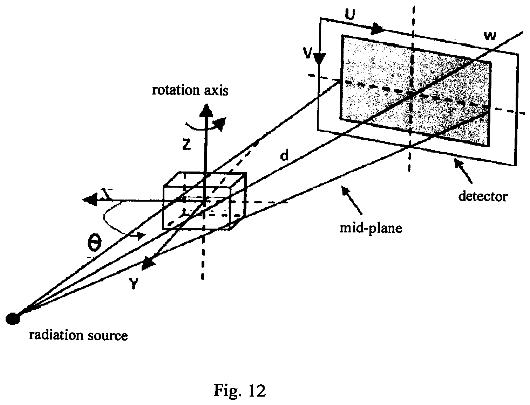

Specifically, the rotation center may be moved to the center of the position corresponding to the area mentioned above. Values corresponding to the position on X direction of the rotation center, the position on Y direction of the rotation center, and the height range of photographing view on Z direction are determined respectively according to XYZ coordinate values corresponding to the area in a preset coordinate system. The preset coordinate system is shown as Fig. 12, and a coordinate center of the preset coordinate system is the center of the position corresponding to the area. The control module in the computed tomography apparatus is configured to control the rotation center of the rotation arm of the computed tomography apparatus to move to the rotation center limited by the XYZ coordinate values, and to control the apparatus to rotate according to a preset rotating angle to capture the area to be captured of the object. It should be understood that the preset rotating angle is at least 180°. The larger the rotating angle, the more image data sources and the better image. Preferably, in the embodiments of the present disclosure, the preset rotating angle is 360°.

It should be noted that, in the embodiments of the present disclosure, the computed tomography apparatus may include the radiation source window, the drive device and the baffle.

Alternatively, in the embodiments of the present disclosure, if the area of the object to be captured determined by the selecting instruction is the partial area of the object to be captured, then the computed tomography apparatus performs photographing on a partial area of the object to be captured according to the position on X direction of the rotation center, the position on Y direction of the rotation center, and the height range of photographing view on Z direction which correspond to the partial area. Specifically, when the area of the object to be captured determined by the selecting instruction is the partial area of the object to be captured, the computed tomography apparatus may control the rotation center of the rotation arm of the computed tomography apparatus to move to the center of the area to be captured (i.e., the partial area mentioned above), and photographing is performed on the partial area via the through-hole by driving the baffle by the drive device to move to the radiation source window such that the through-hole in the baffle is located in the center of the radiation source window according to the position on X direction of the rotation center, the position on Y direction of the rotation center, and the height range of photographing view on Z direction which correspond to the partial area. In other words, the photographing for a partial area is performed by allowing the X rays passing through the radiation source window, penetrating through the through-hole and the partial area mentioned above and emitted on the area array detector.

Alternatively, in the embodiments of the present disclosure, if the area of the object to be captured determined by the selecting instruction is the whole area of the object to be captured, then the computed tomography apparatus performs photographing on a whole area of the object to be captured according to the position on X direction of the rotation center, the position on Y direction of the rotation center, and the height range of photographing view on Z direction which correspond to the whole area. Specifically, when the area of the object to be captured determined by the selecting instruction is the object to be captured, the computed tomography apparatus may control the rotation center of the rotation arm of the computed tomography apparatus to move to the center of the area to be captured (i.e., the whole area mentioned above), and the photographing is performed on the whole area via the radiation source window by driving the baffle by the drive device away from the radiation source window according to the position on X direction of the rotation center, the position on Y direction, and the height range of photographing view on Z direction. In other words, the photographing for a whole area is performed by allowing the X rays passing through the radiation source window and the whole area mentioned above and emitted on the area array detector. In the embodiment of the present disclosure, the drive device drives the baffle away from the radiation source window, which could be understood that the baffle may be moved left and right by the drive device such that the baffle doesn't cover the radiation source window.

Further, in the embodiment of the present disclosure, after the photographing is performed on the partial area, the method also includes: cropping the projection image captured to acquire a partial projection image corresponding to the partial area of the object to be captured. For example, as shown in Fig. 9, the size of the area array detector used in CT photographing for a whole area is ad, and only area be in the image is required to use for performing CT photographing on a partial area, and other areas in the image are unavailable due to a coverage of the window. Thus, when the CT photographing for a partial area is performed, the image may be cropped into a be area image according to geometric size.

In act S504, a captured projection image is acquired, and a CT reconstruction is performed on the projection image according to a preset CT reconstruction model to generate a three-dimensional image.

In the embodiments of the present disclosure, the preset CT reconstruction model is: $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ wherein *f*(*x,y,z*) is the three-dimensional image, *x* is a value corresponding to the rotation center on X direction, y is a value corresponding to the rotation center on Y direction, z is a value corresponding to the height range of photographing view on Z direction, h is an impulse response of a filter in the computed tomography apparatus, cos(*γ*) is cosine of an included angle between a line joining a radiation source to the rotation center and a line joining the radiation source to a point to be measured, *P̃_{θ}*(*u,v*) is weighted filtered data, *d* is a distance between the radiation source and the rotation center, *θ* is an angle of the projection image, *u* is an abscissa value corresponding to the projection image, v is an ordinate value corresponding to the projection image, *α*₁ is a starting angle of the projection image and *α*₂ is an ending angle of the projection image.

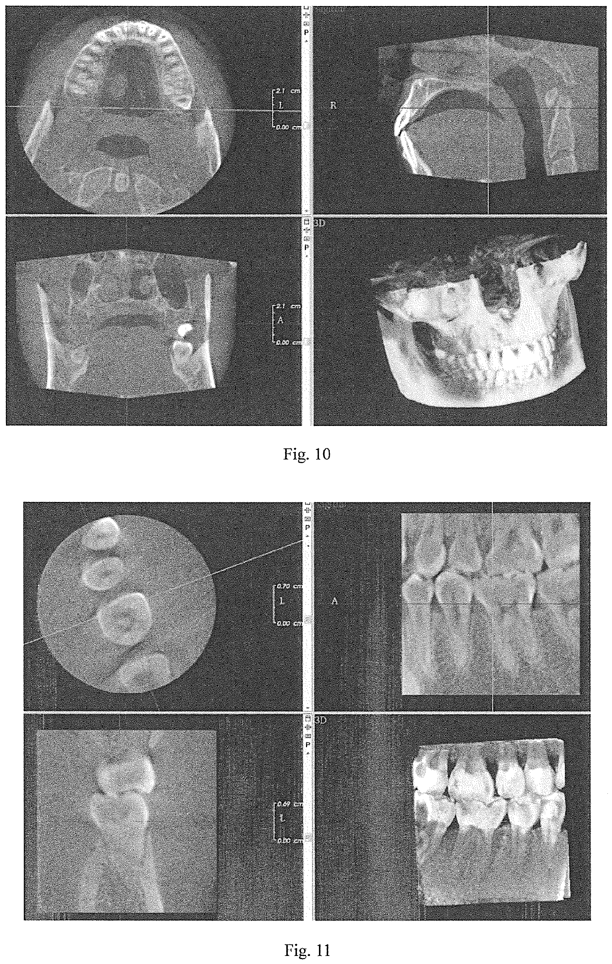

Specifically, if the CT photographing for a whole area is performed, a captured full view projection image may be acquired, and the CT reconstruction may be performed on the full view projection image according to the preset CT reconstruction model to generate a full view three-dimensional image (for example, a reconstructed full view three-dimensional image when the CT photographing for a whole area is performed is shown in Fig. 10). If the CT photographing for a partial area is performed, a cropped partial view projection image may be acquired, and the CT reconstruction may be performed on the partial view projection image according to the preset CT reconstruction model to generate a partial view three-dimensional image (for example, a reconstructed partial view three-dimensional image when the CT photographing for a partial area is performed is shown in Fig. 11).

It should be understood that a principle of the partial CT reconstruction is similar to that of the CT reconstruction, and the partial CT is just treated as a small view CT of which the size of the captured image is smaller and pixel points are less, thus improving resolution of the partial image.

A process for acquiring the preset CT reconstruction model will be described in detail as follows.

The process for perform the CT photographing is illustrated in Fig. 12. The radiation source and the detector rotate 360°around a rotation axis (Z). In this rotating process, the detector collects images with a fixed frame frequency.

Assume that *pq(u,v)* represents the projection image acquired after photographing, which means that projection data received by the detector when the radiation source rotates at an angle *q. W* represents a bandwidth of an actual signal.

Firstly, *Qϑ*(*u,v*) representing weighted projection data is acquired by multiplying the projection data by cosine of an included angle between a line joining a radiation source to the rotation center and the line joining the radiation source to a point to be measured, and then *P̃_{θ}*(*u,v*) representing weighted filtering data by performing a convolution on the weighted projection data *Q_{ϑ}*(*u,v*) and a filter with impulse response *h,* i.e., *P̃₀*(*u,v*) = *Q_{θ}*(*u,v*)^{∗}*h*(*v*), in which ${Q}_{θ} \left(u, v\right) = {p}_{θ} \left(u, v\right) . cos \left(γ\right) , cos \left(γ\right) = \frac{d + h - z}{\sqrt{{x}^{2} + {y}^{2} + {\left(z-d-h\right)}^{2}}}$ $u = \frac{\left(d+h\right) \left(xsinθ-zcosθ\right)}{\left(d+h\right) sin θ - z} , v = \frac{y \left(d+h\right) sin θ}{\left(d+h\right) sin θ - z}$ $h \left(t\right) = {\int }_{- W}^{W} {e}^{jwt} \left|w\right| dw .$

Finally, *f*(*x,y,z*) representing a reconstruction image data is acquired by performing a weighted back projection on the weighted filtering data *P̃_{θ}*(*u*,*v*), in which $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ .$

It could be seen that the weighting process above shows influence of different distances between the object and the radiation source on the projection data acquired in cone-beam imaging.

In conclusion, there are at least following advantages of the method for capturing a tomographic image by the computed tomography apparatus according to any of embodiments of the present disclosure.
(1) Image resolution is improved.
(2) Radiation dosage may be reduced, for which the reason is that a window of a beam limiting device is larger since a field of view of the CT photographing for a whole area is larger. For example, the size of a CT window is required to be 28 mm ^{∗} 28 mm, while the size of a partial CT window is only required to be 14 mm ^{∗} 14 mm. Thus, from a perspective of the window, radiation dosage for the partial CT is 3/4 lesser than CT (the size of the partial CT window is determined according to the size of the area to be captured, and it is only an example mentioned above). Thus, it could be seen that radiation dosage for the partial CT is reduced compared to that for the photographing for a whole area.
(3) Image resolution is improved and a time for reconstructing is reduced in a case of same collecting data as CT. The target object may be recovered as a combination of voxels by performing a CT reconstruction, in which each small voxel has a value. For example, a size of one reconstructed image is 672 ^{∗} 672, and there are a total of 448 images, which could be understood that there are 672 ^{∗} 672 ^{∗} 448 small voxels (672 voxels on X direction, 672 voxels on Y direction, and 448 voxels on Z direction), and a result of the reconstruction is giving each small voxel a value of its own.
   Reconstruction time is longer and computer resources used is more in the CT reconstruction due to a massive computation for the CT reconstruction. For example, the size of one reconstructed image is 672 ^{∗} 672, there are a total of 448 images, and resolution may reach 0.19 mm (in which a field of view of the image reaches a size that a diameter on an XY plane is 672 ^{∗} 0.19 = 127.68 mm, and a height on Z direction is 448 ^{∗} 0.19 = 85.12 mm). A memory space is required to be 672 ^{∗} 672 ^{∗} 448 ^{∗} 4 = 809238528 bits, in which one float datum needs 4 bits. However, it is merely an upper limit for a common configuration of a computer (with a 1∼2 GB video card and a 4 GB memory). Unless a high performance computing computer is used or there may be a massive computation load to improve resolution and to increase the size of the image.
   Since a size of an area to be reconstructed when a partial CT reconstruction is performed is far less than that of a CT area, the constructing time may be saved and the image resolution may be improved as well. For example, the size of one image after a location reconstruction is 480 ^{∗} 480, there are a total of 448 images, and resolution may reach 0.07 mm (in which a field of view of the image reaches a size that a diameter on an XY plane is 480 ^{∗} 0.07 = 33.6 mm, and a height on Z direction is 480 ^{∗} 0.07 = 33.6 mm). Thus, resolution of the partial CT image is higher than that of the CT image under a same condition, which is useful for a doctor when a single tooth and an around condition thereof are required to be observed.
(4) An upgrade may be performed directly on an oral CT machine by upgrading additional functions by software without replacing other hardware.
(5) A collecting time is reduced. A minimum rotation angle for collecting under a CT mode is required to be larger than 180° if the detector is deviated, and a 180° angle is enough for the partial CT collection if a field of view of the partial CT is detected by the detector totally, thus the collecting time is reduced.

According to the method of the embodiments of the present disclosure, the selecting instruction for the pre-captured image may be received from the user, and the area of the object to be captured is determined according to the selecting instruction, the position on X direction and the position on Y direction of the rotation center of the rotation arm in the computed tomography apparatus and the height range of photographing view on Z direction are determined according to the area, such that the computed tomography apparatus captures the area according to the position on X direction and the position on Y direction of the rotation center, and the height range of photographing view on Z direction, the projection image captured is acquired, and the CT reconstruction is performed on the projection image according to the preset CT reconstruction model to generate the three-dimensional image. For example, when the user only needs to observe the partial area of the object to be captured, the baffle can be regulated by the computed tomography apparatus to realize the CT photographing for a partial area on the target object. Further, for example, when the user only needs to observe the whole area of the object to be captured, the baffle is regulated by the computed tomography apparatus to realize the CT photographing for a whole area on the target object, i.e., the baffle is regulated by the computed tomography apparatus to switch between the CT photographing for a partial area and the CT photographing for a whole area.

In the specification, it is to be understood that terms such as "central," "longitudinal," "lateral," "length," "width," "thickness," "upper," "lower," "front," "rear," "left," "right," "vertical," "horizontal," "top," "bottom," "inner," "outer," "clockwise," and "counterclockwise" should be construed to refer to the orientation as then described or as shown in the drawings under discussion. These relative terms are for convenience of description and do not require that the present invention be constructed or operated in a particular orientation.

In the present invention, unless specified or limited otherwise, the terms "mounted," "connected," "coupled," "fixed" and the like are used broadly, and may be, for example, fixed connections, detachable connections, or integral connections; may also be mechanical or electrical connections; may also be direct connections or indirect connections via intervening structures; may also be inner communications of two elements, which can be understood by those skilled in the art according to specific situations.

Reference throughout this specification to "one embodiment", "some embodiments," "an embodiment" , "a specific example," or "some examples," means that a particular feature, structure, material, or characteristic described in connection with the embodiment or example is included in at least one embodiment or example of the present disclosure. Thus, the appearances of the phrases in various places throughout this specification are not necessarily referring to the same embodiment or example of the present disclosure. Furthermore, the particular features, structures, materials, or characteristics may be combined in any suitable manner in one or more embodiments or examples. In addition, in a case without contradictions, different embodiments or examples or features of different embodiments or examples may be combined by those skilled in the art.

## Claims

1. A method for capturing a tomographic image by a computed tomography apparatus, the computer tomography apparatus comprising:
a radiation source, configured to emit X rays;
a radiation source window;
a baffle, having a through-hole therein;
a control module, connected to the baffle via a drive device, configured to control a rotation center of a rotation arm of the computed tomography apparatus according to a preset photographing condition to determine a photographing position to be captured, and to regulate the baffle via the drive device according to a position of a partial area of a target object when the photographing position is the partial area of the target object, such that the X rays pass through the radiation source window and penetrate through the through-hole and the partial area to an area array detector; and
the area array detector, configured to convert received X rays penetrating through the through-hole and the partial area to a projection image;
the method comprising:
acquiring a pre-captured image (501);
receiving a selecting instruction for the pre-captured image from a user, and determining an area of an object to be captured according to the selecting instruction (502);
determining a position on X direction of the rotation center, a position on Y direction of the rotation center, and a height range of photographing view on Z direction according to the area, such that the computed tomography apparatus captures the area according to the position on X direction, the position on Y direction, and the height range of photographing view on Z direction (503); and
acquiring a projection image captured, and performing a CT reconstruction on the projection image according to a preset CT reconstruction model to generate a three-dimensional image (504),
**characterised in that** the preset CT reconstruction model is: $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ where, *f*(*x,y, z)* is the three-dimensional image, *x* is a value corresponding to the rotation center on X direction, y is a value corresponding to the rotation center on Y direction, z is a value corresponding to the height range of photographing view on Z direction, h is an impulse response of a filter in the computed tomography apparatus, cos(*γ*) is cosine of an included angle between a line joining the radiation source (10) to the rotation center and a line joining the radiation source (10) to a point to be measured, *P_{θ}*(*u,v*) is weighted filtered data, *d* is a distance between the radiation source (10) and the rotation center, *θ* is an angle of the projection image, u is an abscissa value corresponding to the projection image, v is an ordinate value corresponding to the projection image, *α*₁ is a starting angle of the projection image, and *α*₂ is an ending angle of the projection image.

2. The method according to claim 1, wherein,
if the area of the object to be captured determined according to the selecting instruction is the partial area of the object to be captured, then the computed tomography apparatus performs a photographing on a partial area of the object to be captured according to the position on X direction of the rotation center, the position on Y direction of the rotation center, and the height range of photographing view on Z direction, corresponding to the partial area; and
if the area of the object to be captured determined according to the selecting instruction is the whole area of the object to be captured, then the computed tomography apparatus performs a photographing on a whole area of the object to be captured according to the position on X direction of the rotation center, the position on Y direction of the rotation center, and the height range of photographing view on Z direction corresponding to the whole area.

3. The method according to claim 2, wherein after the computed tomography apparatus performs a photographing on a partial area, the method further comprises:
cropping the projection image captured to acquire a partial projection image corresponding to the partial area of the object to be captured.

4. A non-transitory computer readable storage medium having stored therein instructions that, when executed by a processor of a computer tomography apparatus comprising:
a radiation source, configured to emit X rays;
a radiation source window;
a baffle, having a through-hole therein;
a control module, connected to the baffle via a drive device, configured to control a rotation center of a rotation arm of the computed tomography apparatus according to a preset photographing condition to determine a photographing position to be captured, and to regulate the baffle via the drive device according to a position of a partial area of a target object when the photographing position is the partial area of the target object, such that the X rays pass through the radiation source window and penetrate through the through-hole and the partial area to an area array detector; and
the area array detector, configured to convert received X rays penetrating through the through-hole and the partial area to a projection image, causes the computer tomography apparatus to perform a method for capturing a tomographic image, wherein the method comprises:
acquiring a pre-captured image;
receiving a selecting instruction for the pre-captured image from a user, and determining an area of an object to be captured according to the selecting instruction;
determining a position on X direction of the rotation center, a position on Y direction of the rotation center, and a height range of photographing view on Z direction according to the area, such that the computed tomography apparatus captures the area according to the position on X direction, the position on Y direction, and the height range of photographing view on Z direction; and
acquiring a projection image captured, and performing a CT reconstruction on the projection image according to a preset CT reconstruction model to generate a three-dimensional image,
**characterised in that** the preset CT reconstruction model is: $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ where, *f*(*x,y,z*) is the three-dimensional image, *x* is a value corresponding to the rotation center on X direction, y is a value corresponding to the rotation center on Y direction, z is a value corresponding to the height range of photographing view on Z direction, h is an impulse response of a filter in the computed tomography apparatus, cos(*γ*) is cosine of an included angle between a line joining the radiation source (10) to the rotation center and a line joining the radiation source (10) to a point to be measured, *P_{θ}*(*u,v*) is weighted filtered data, *d* is a distance between the radiation source (10) and the rotation center, *θ* is an angle of the projection image, u is an abscissa value corresponding to the projection image, v is an ordinate value corresponding to the projection image, *α*₁ is a starting angle of the projection image, and *α*₂ is an ending angle of the projection image.

5. The non-transitory computer readable storage medium according to claim 4, wherein the method further comprises:
if the area of the object to be captured determined according to the selecting instruction is the partial area of the object to be captured, performing a photographing on a partial area of the object to be captured according to the position on X direction of the rotation center, the position on Y direction of the rotation center, and the height range of photographing view on Z direction, corresponding to the partial area;
if the area of the object to be captured determined according to the selecting instruction is the whole area of the object to be captured, performing a photographing on a whole area of the object to be captured according to the position on X direction of the rotation center, the position on Y direction of the rotation center, and the height range of photographing view on Z direction corresponding to the whole area.

6. The non-transitory computer readable storage medium according to claim 5, wherein after performing a photographing on a partial area of the object to be captured according to the position on X direction of the rotation center, the position on Y direction of the rotation center, and the height range of photographing view on Z direction, corresponding to the partial area, the method further comprises:
cropping the projection image captured to acquire a partial projection image corresponding to the partial area of the object to be captured.

## Patentansprüche

1. Verfahren zur Aufnahme eines tomographischen Bilds mittels einer Computertomographievorrichtung, wobei die Computertomographievorrichtung Folgendes umfasst:
eine Strahlungsquelle zur Abgabe von Röntgenstrahlen;
ein Strahlungsquellenfenster;
eine Blende mit einer darin ausgebildeten Durchgangsöffnung;
ein Steuermodul, das über eine Antriebsvorrichtung mit der Blende verbunden und dazu ausgebildet ist, einen Drehpunkt eines Rotationsarms der Computertomographievorrichtung gemäß einer vorbestimmten Bildaufnahmebedingung zu steuern, um eine aufzunehmende Bildaufnahmeposition zu bestimmen, und die Blende über die Antriebsvorrichtung gemäß einer Position eines Teilbereichs eines Zielobjekts so zu steuern, wenn es sich bei der Bildaufnahmeposition um den Teilbereich des Zielobjekts handelt, dass die Röntgenstrahlen das Strahlenquellenfenster durchqueren und durch das Durchgangsloch und den Teilbereich hindurch zu einem Flächendetektor gelangen; und
den Flächendetektor, der zur Umwandlung von durch das Durchgangsloch und den Teilbereich hindurch empfangenen Röntgenstrahlen in ein Projektionsbild ausgebildet ist;
wobei das Verfahren Folgendes umfasst:
Erstellen eines voraufgenommenen Bildes (501);
Empfangen eines Auswahlbefehls für das voraufgenommene Bild von einem Nutzer und Bestimmen eines Bereichs eines aufzunehmenden Objekts gemäß dem Auswahlbefehl (502);
Bestimmen einer Position auf einer X-Achse des Drehpunkts, einer Position auf einer Y-Achse des Drehpunkts und eines Höhenbereichs des Bildaufnahmesichtfelds auf einer Z-Achse gemäß dem Bereich, so dass die Computertomographievorrichtung den Bereich gemäß der Position auf der X-Achse, der Position auf der Y-Achse und dem Höhenbereich des Bildaufnahmesichtfelds auf der Z-Achse aufnimmt (503); und
Erstellen eines aufgenommenen Projektionsbilds und Durchführen einer CT-Rekonstruktion an dem Projektionsbild gemäß einem vorbestimmten CT-Rekonstruktionsmodell, um ein dreidimensionales Bild zu erzeugen (504),
**dadurch gekennzeichnet,**
**dass** das vorbestimmte CT-Rekonstruktionsmodell wie folgt lautet: $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ wobei *f*(*x,y,z*) das dreidimensionale Bild ist, *x* ein dem Drehpunkt entsprechender Wert auf der X-Achse ist, y ein dem Drehpunkt entsprechender Wert auf der Y-Achse ist, z ein dem Höhenbereich des Bildaufnahmesichtfelds entsprechender Wert auf der Z-Achse ist, *h* eine Impulsantwort eines Filters in der Computertomographievorrichtung ist, cos(*γ*) ein Kosinus eines Winkels ist, der zwischen einer die Strahlungsquelle (10) mit dem Drehpunkt verbindenden Linie und einer die Strahlungsquelle (10) mit einem Messpunkt verbindenden Linie aufgespannt ist, *P̃_{θ}*(*u,v*) gewichtete Filterdaten sind, *d* ein Abstand zwischen der Strahlungsquelle (10) und dem Drehpunkt ist, *θ* ein Winkel des Projektionsbilds ist, u ein dem Projektionsbild entsprechender Abszissenwert ist, v ein dem Projektionsbild entsprechender Ordinatenwert ist, *α*₁ ein Ausgangswinkel des Projektionsbilds ist und *α*₂ ein Endwinkel des Projektionsbilds ist.

2. Verfahren nach Anspruch 1, wobei,
wenn der Bereich des aufzunehmenden Objekts, der gemäß dem Auswahlbefehl bestimmt wurde, der Teilbereich des aufzunehmenden Objekts ist, die Computertomographievorrichtung eine Bildaufnahme eines Teilbereichs des aufzunehmenden Objekts gemäß der dem Teilbereich entsprechenden Position auf der X-Achse des Drehpunkts, der dem Teilbereich entsprechenden Position auf der Y-Achse des Drehpunkts und dem dem Teilbereich entsprechenden Höhenbereich des Bildaufnahmesichtfelds auf der Z-Achse vornimmt; und
wenn der Bereich des aufzunehmenden Objekts, der gemäß dem Auswahlbefehl bestimmt wurde, der gesamte Bereich des aufzunehmenden Objekts ist, die Computertomographievorrichtung eine Bildaufnahme des gesamten Bereichs des aufzunehmenden Objekts gemäß der dem gesamten Bereich entsprechenden Position auf der X-Achse des Drehpunkts, der dem gesamten Bereich entsprechenden Position auf der Y-Achse des Drehpunkts und dem dem gesamten Bereich entsprechenden Höhenbereich des Bildaufnahmesichtfelds auf der Z-Achse vornimmt.

3. Verfahren nach Anspruch 2, wobei das Verfahren nach dem Durchführen einer Bildaufnahme eines Teilbereichs durch die Computertomographievorrichtung des Weiteren Folgendes umfasst:
Zuschneiden des aufgenommenen Projektionsbilds zur Erstellung eines dem Teilbereich des aufzunehmenden Objekts entsprechenden Teilprojektionsbilds.

4. Nicht flüchtiges computerlesbares Speichermedium, auf welchem Befehle gespeichert sind, deren Ausführung durch einen Prozessor einer Computertomographievorrichtung, die Folgendes umfasst:
eine Strahlungsquelle zur Abgabe von Röntgenstrahlen;
ein Strahlungsquellenfenster;
eine Blende mit einer darin ausgebildeten Durchgangsöffnung;
ein Steuermodul, das über eine Antriebsvorrichtung mit der Blende verbunden und dazu ausgebildet ist, einen Drehpunkt eines Rotationsarms der Computertomographievorrichtung gemäß einer vorbestimmten Bildaufnahmebedingung zu steuern, um eine aufzunehmende Bildaufnahmeposition zu bestimmen, und die Blende über die Antriebsvorrichtung gemäß einer Position eines Teilbereichs eines Zielobjekts so zu steuern, wenn es sich bei der Bildaufnahmeposition um den Teilbereich des Zielobjekts handelt, dass die Röntgenstrahlen das Strahlenquellenfenster durchqueren und durch das Durchgangsloch und den Teilbereich hindurch zu einem Flächendetektor gelangen; und
den Flächendetektor, der zur Umwandlung von durch das Durchgangsloch und den Teilbereich hindurch empfangenen Röntgenstrahlen in ein Projektionsbild ausgebildet ist,
die Computertomographievorrichtung dazu veranlasst, ein Verfahren zur Aufnahme eines tomographischen Bilds durchzuführen, wobei das Verfahren Folgendes umfasst:
Erstellen eines voraufgenommenen Bildes (501);
Empfangen eines Auswahlbefehls für das voraufgenommene Bild von einem Nutzer und Bestimmen eines Bereichs eines aufzunehmenden Objekts gemäß dem Auswahlbefehl;
Bestimmen einer Position auf einer X-Achse des Drehpunkts, einer Position auf einer Y-Achse des Drehpunkts und eines Höhenbereichs des Bildaufnahmesichtfelds auf einer Z-Achse gemäß dem Bereich, so dass die Computertomographievorrichtung den Bereich gemäß der Position auf der X-Achse, der Position auf der Y-Achse und dem Höhenbereich des Bildaufnahmesichtfelds auf der Z-Achse aufnimmt; und
Erstellen eines aufgenommenen Projektionsbilds und Durchführen einer CT-Rekonstruktion an dem Projektionsbild gemäß einem vorbestimmten CT-Rekonstruktionsmodell, um ein dreidimensionales Bild zu erzeugen,
**dadurch gekennzeichnet,**
**dass** das vorbestimmte CT-Rekonstruktionsmodell wie folgt lautet: $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ wobei *f*(*x,y,z*) das dreidimensionale Bild ist, x ein dem Drehpunkt entsprechender Wert auf der X-Achse ist, *y* ein dem Drehpunkt entsprechender Wert auf der Y-Achse ist, z ein dem Höhenbereich des Bildaufnahmesichtfelds entsprechender Wert auf der Z-Achse ist, h eine Impulsantwort eines Filters in der Computertomographievorrichtung ist, cos(*γ*) ein Kosinus eines Winkels ist, der zwischen einer die Strahlungsquelle (10) mit dem Drehpunkt verbindenden Linie und einer die Strahlungsquelle (10) mit einem Messpunkt verbindenden Linie aufgespannt ist, *P̃_{θ}*(*u,v*) gewichtete Filterdaten sind, *d* ein Abstand zwischen der Strahlungsquelle (10) und dem Drehpunkt ist, *θ* ein Winkel des Projektionsbilds ist, u ein dem Projektionsbild entsprechender Abszissenwert ist, v ein dem Projektionsbild entsprechender Ordinatenwert ist, *α*₁ ein Ausgangswinkel des Projektionsbilds ist und *α*₂ ein Endwinkel des Projektionsbilds ist.

5. Nicht flüchtiges computerlesbares Speichermedium nach Anspruch 4, wobei das Verfahren des Weiteren Folgendes umfasst:
wenn der Bereich des aufzunehmenden Objekts, der gemäß dem Auswahlbefehl bestimmt wurde, der Teilbereich des aufzunehmenden Objekts ist, Durchführen einer Bildaufnahme eines Teilbereichs des aufzunehmenden Objekts gemäß der dem Teilbereich entsprechenden Position auf der X-Achse des Drehpunkts, der dem Teilbereich entsprechenden Position auf der Y-Achse des Drehpunkts und dem dem Teilbereich entsprechenden Höhenbereich des Bildaufnahmesichtfelds auf der Z-Achse; und
wenn der Bereich des aufzunehmenden Objekts, der gemäß dem Auswahlbefehl bestimmt wurde, der gesamte Bereich des aufzunehmenden Objekts ist, Durchführen einer Bildaufnahme des gesamten Bereichs des aufzunehmenden Objekts gemäß der dem gesamten Bereich entsprechenden Position auf der X-Achse des Drehpunkts, der dem gesamten Bereich entsprechenden Position auf der Y-Achse des Drehpunkts und dem dem gesamten Bereich entsprechenden Höhenbereich des Bildaufnahmesichtfelds auf der Z-Achse.

6. Nicht flüchtiges computerlesbares Speichermedium nach Anspruch 5, wobei das Verfahren nach dem Durchführen einer Bildaufnahme eines Teilbereichs des aufzunehmenden Objekts gemäß der dem Teilbereich entsprechenden Position auf der X-Achse des Drehpunkts, der dem Teilbereich entsprechenden Position auf der Y-Achse des Drehpunkts und dem dem Teilbereich entsprechenden Höhenbereich des Bildaufnahmesichtfelds auf der Z-Achse des Weiteren Folgendes umfasst:
Zuschneiden des aufgenommenen Projektionsbilds zur Erstellung eines dem Teilbereich des aufzunehmenden Objekts entsprechenden Teilprojektionsbilds.

## Revendications

1. Procédé de prise d'une image tomographique par un dispositif de tomographie assisté par ordinateur (CT), le dispositif de tomographie assisté par ordinateur comprenant :
une source de rayonnement configurée pour émettre des rayons X ;
une fenêtre de source de rayonnement ;
un déflecteur ayant un trou de passage ;
un module de commande connecté au déflecteur par l'intermédiaire d'un dispositif d'entraînement et configuré pour commander un centre de rotation d'un bras de rotation du dispositif de tomographie assisté par ordinateur conformément à une condition de prise de vue prédéfinie afin de déterminer une position de prise de vue à laquelle une image doit être prise, et pour réguler le déflecteur par l'intermédiaire du dispositif d'entraînement conformément à une position d'une zone partielle d'un objet cible lorsque la position de prise de vue est la zone partielle de l'objet cible, de telle manière que les rayons X traversent la fenêtre de source de rayonnement et pénètrent à travers le trou de passage et la zone partielle jusqu'à un détecteur à réseau plan ; et
le détecteur à réseau plan configuré pour convertir des rayons X reçus pénétrant à travers le trou de passage et la zone partielle en une image de projection ;
le procédé comprenant des étapes consistant à :
acquérir une image pré-prise (501) ;
recevoir une instruction de sélection pour l'image pré-prise d'un utilisateur et déterminer une zone d'un objet dont une image doit être prise conformément à l'instruction de sélection (502) ;
déterminer une position dans une direction X du centre de rotation, une position dans une direction Y du centre de rotation et une gamme de hauteur de la vue de prise de vue dans une direction Z conformément à la zone de telle manière que le dispositif de tomographie assisté par ordinateur prend une image de la zone conformément à la position dans la direction X, à la position dans la direction Y et à la gamme de hauteur de la vue de la prise de vue dans la direction Z (503) ; et
acquérir une image de projection prise et effectuer une reconstruction CT sur l'image de projection conformément à un modèle de reconstruction CT prédéfini afin de générer une image tridimensionnelle (504),
**caractérisé en ce que**
le modèle de reconstruction CT prédéfini est : $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ dans lequel *f*(*x,y,z*) est l'image tridimensionnelle, *x* est une valeur correspondant au centre de rotation dans la direction X, *y* est une valeur correspondant au centre de rotation dans la direction Y, z est une valeur correspondant à la gamme de hauteur de la vue de la prise de vue dans la direction Z, *h* est une réponse impulsionnelle d'un filtre dans le dispositif de tomographie assisté par ordinateur, cos(*γ*) est un cosinus d'un angle inclus entre une ligne reliant la source de rayonnement (10) et le centre de rotation et une ligne reliant la source de rayonnement (10) et un point à mesurer, *P̃_{θ}*(*u,v*) sont des données de filtre pondérées, *d* est une distance entre la source de rayonnement (10) et le centre de rotation, *θ* est un angle de l'image de projection, u est une valeur d'abscisse correspondant à l'image de projection, v est une valeur d'ordonnée correspondant à l'image de projection, *α*₁ est un angle initial de l'image de projection et *α*₂ est un angle final de l'image de projection.

2. Procédé selon la revendication 1, dans lequel,
si la zone de l'objet dont une image doit être prise déterminée conformément à l'instruction de sélection est la zone partielle de l'objet dont une image doit être prise, le dispositif de tomographie assisté par ordinateur effectue une prise de vue d'une zone partielle de l'objet dont une image doit être prise conformément à la position dans la direction X du centre de rotation, à la position dans la direction Y du centre de rotation et à la gamme de hauteur de la vue de la prise de vue dans la direction Z, correspondant à la zone partielle ; et
si la zone de l'objet dont une image doit être prise déterminée conformément à l'instruction de sélection est la zone entière de l'objet dont une image doit être prise, le dispositif de tomographie assisté par ordinateur effectue une prise de vue d'une zone entière de l'objet dont une image doit être prise conformément à la position dans la direction X du centre de rotation, à la position dans la direction Y du centre de rotation et à la gamme de hauteur de la vue de la prise de vue dans la direction Z, correspondant à la zone entière.

3. Procédé selon la revendication 2, dans lequel, après le dispositif de tomographie assisté par ordinateur a effectué une prise de vue d'une zone partielle, le procédé comprend en outre une étape consistant à :
recadrer l'image de projection prise afin d'acquérir une image de projection partielle correspondant à la zone partielle de l'objet dont une image doit être prise.

4. Support de stockage non transitoire lisible par ordinateur, le support de stockage ayant stocké dans celui-ci des instructions qui, lorsqu'elles sont exécutées par un processeur d'un dispositif de tomographie assisté par ordinateur (CT) comprenant :
une source de rayonnement configurée pour émettre des rayons X ;
une fenêtre de source de rayonnement ;
un déflecteur ayant un trou de passage ;
un module de commande connecté au déflecteur par l'intermédiaire d'un dispositif d'entraînement et configuré pour commander un centre de rotation d'un bras de rotation du dispositif de tomographie assisté par ordinateur conformément à une condition de prise de vue prédéfinie afin de déterminer une position de prise de vue à laquelle une image doit être prise, et pour réguler le déflecteur par l'intermédiaire du dispositif d'entraînement conformément à une position d'une zone partielle d'un objet cible lorsque la position de prise de vue est la zone partielle de l'objet cible, de telle manière que les rayons X traversent la fenêtre de source de rayonnement et pénètrent à travers le trou de passage et la zone partielle jusqu'à un détecteur à réseau plan ; et
le détecteur à réseau plan configuré pour convertir des rayons X reçus pénétrant à travers le trou de passage et la zone partielle en une image de projection,
amènent le dispositif de tomographie assisté par ordinateur à effectuer un procédé de prise d'une image tomographique, dans lequel le procédé comprend des étapes consistant à :
acquérir une image pré-prise ;
recevoir une instruction de sélection pour l'image pré-prise d'un utilisateur et déterminer une zone d'un objet dont une image doit être prise conformément à l'instruction de sélection ;
déterminer une position dans une direction X du centre de rotation, une position dans une direction Y du centre de rotation et une gamme de hauteur de la vue de la prise de vue dans une direction Z conformément à la zone de telle manière que le dispositif de tomographie assisté par ordinateur prend une image de la zone conformément à la position dans la direction X, à la position dans la direction Y et à la gamme de hauteur de la vue de la prise de vue dans la direction Z ; et
acquérir une image de projection prise et effectuer une reconstruction CT sur l'image de projection conformément à un modèle de reconstruction CT prédéfini afin de générer une image tridimensionnelle,
**caractérisé en ce que**
le modèle de reconstruction CT prédéfini est : $f \left(x, y, z\right) = \frac{1}{4 {π}^{2}} {\int }_{{α}_{1}}^{{α}_{2}} \frac{{d}^{2}}{\left({x}^{2}+{y}^{2}+{\left(z-d-h\right)}^{2}\right) {cos}^{2} γ} {{P}^{˜}}_{θ} \left(u, v\right) dθ ,$ dans lequel *f*(*x,y,z*) est l'image tridimensionnelle, *x* est une valeur correspondant au centre de rotation dans la direction X, *y* est une valeur correspondant au centre de rotation dans la direction Y, z est une valeur correspondant à la gamme de hauteur de la vue de la prise de vue dans la direction Z, *h* est une réponse impulsionnelle d'un filtre dans le dispositif de tomographie assisté par ordinateur, cos(*γ*) est un cosinus d'un angle inclus entre une ligne reliant la source de rayonnement (10) et le centre de rotation et une ligne reliant la source de rayonnement (10) et un point à mesurer, *P̃_{θ}*(*u,v*) sont des données de filtre pondérées, *d* est une distance entre la source de rayonnement (10) et le centre de rotation, *θ* est un angle de l'image de projection, u est une valeur d'abscisse correspondant à l'image de projection, v est une valeur d'ordonnée correspondant à l'image de projection, *α*₁ est un angle initial de l'image de projection et *α*₂ est un angle final de l'image de projection.

5. Support de stockage non transitoire lisible par ordinateur selon la revendication 4, dans lequel le procédé comprend en outre des étapes consistant à :
si la zone de l'objet dont une image doit être prise déterminée conformément à l'instruction de sélection est la zone partielle de l'objet dont une image doit être prise, effectuer une prise de vue d'une zone partielle de l'objet dont une image doit être prise conformément à la position dans la direction X du centre de rotation, à la position dans la direction Y du centre de rotation et à la gamme de hauteur de la vue de la prise de vue dans la direction Z, correspondant à la zone partielle ; et
si la zone de l'objet dont une image doit être prise déterminée conformément à l'instruction de sélection est la zone entière de l'objet dont une image doit être prise, effectuer une prise de vue d'une zone entière de l'objet dont une image doit être prise conformément à la position dans la direction X du centre de rotation, à la position dans la direction Y du centre de rotation et à la gamme de hauteur de la vue de la prise de vue dans la direction Z, correspondant à la zone entière.

6. Support de stockage non transitoire lisible par ordinateur selon la revendication 5, dans lequel, après qu'une prise de vue d'une zone partielle de l'objet dont une image doit être prise a été effectuée conformément à la position dans la direction X du centre de rotation, à la position dans la direction Y du centre de rotation et à la gamme de hauteur de la vue de la prise de vue dans la direction Z, correspondant à la zone partielle, le procédé comprend en outre une étape consistant à :
recadrer l'image de projection prise afin d'acquérir une image de projection partielle correspondant à la zone partielle de l'objet dont une image doit être prise.
